# EUROPEAN PATENT APPLICATION

(11) **EP 3 792 863 A1**
(43) Date of publication of application: **17.03.2021**
(21) Application number: 19798881.9
(22) Date of filing: 13.05.2019
(51) Int. Cl.: G06Q 50/12, G16H 20/60, G06Q 50/10

(54) **MODULARIZED MENU PROVIDING SERVICE SYSTEM**

(30) Priority: 11.05.2018 KR 20180054556
(71) Applicant: Yang, Saheon, Gyeonggi-do 10580 (KR)
(72) Inventor: Yang, Saheon, Gyeonggi-do 10580 (KR)
(74) Representative: Peters, Sebastian Martinus
(86) International application number: PCT/KR2019/005744
(87) International publication number: WO 2019/216734

(57) **Abstract**

The present invention relates to a modularized menu providing service system and, more particularly, to a menu providing service system comprising a service providing server for receiving order information from a user device and delivering same to a member store device. The service providing server comprises: a database for storing and managing menu configuration elements comprising modularized food elements constituting a menu, and modularized setting elements; a menu configuration portion for providing the user device with the menu configuration elements stored in the database, receiving a selection of the provided menu configuration elements from the user device, and configuring a menu; a price calculation portion for calculating the price of the selected menu configuration elements with regard to the configured menu; and an ordering portion for receiving order information from the user device and delivering the menu configured by the menu configuration module and the received order information to the member store device. The modularized food elements comprise the type of food, the unit amount of food configured according to the type of food, and the method for providing same. The modularized setting elements comprise setting types comprising packaging, tableware, cooking utensils, heaters, and kitchen cooking. According to the modularized menu providing service system proposed by the present invention, the user device is provided with menu configuration elements comprising modularized food elements and modularized setting elements, and a menu configured according to the user's selection is delivered to a member store as an order. Accordingly, modularized food elements and setting elements can be combined such that food desired by the user can be ordered by a desired amount in a desired manner. In addition, even dishes or cooking tools can be selected by selecting setting elements such as packaging, tableware, and cooking utensils, thereby enabling the user to cook food as desired and to eat same. In addition, according to the present invention, standardized weights and standardized calories are stored and managed according to the unit amount of food, with regard to modularized food elements. Accordingly, the menu and calories of a menu can be provided in a practical manner by using the unit amount of food on the basis of a concept that can be used when a menu is actually provided. In addition, weights and calories are standardized and provided, which are difficult to accurately calculate when a menu is actually provided, thereby improving the level of satisfaction of users interested in diet and health.

## Description

### Technical Field

The present invention relates to a menu providing service system and, more particularly, to a modularized menu providing service system.

### Background Art

Recently, due to time and space limitations, there are increasing numbers of people looking for restaurants or delivery foods near work and home. At the same time, interest in diet and food for health is increasing. That is, while awareness and interest in eating healthy food is increasing, it is difficult to cook food personally due to the rapid increase of single-person households and the hassle of purchasing and cooking ingredients.

In order to solve such a problem, a service has been developed in which a pre-cooked frozen lunch box is delivered and stored according to a pre-configured diet menu, and is heated while being stored, or a food cooked according to the menu is delivered at regular intervals. However, such a lunch box or delivery service cannot cook and eat the desired ingredients in a desired manner as much as desired, and there is a limit in that freshness may be deteriorated during freezing or delivery.

Meanwhile, with the rapid development of mobile devices and communication technologies, it is possible to increase the convenience of life by using applications with various functions installed on mobile devices or mobile webs. Among the applications and mobile webs, some provide menu information and deliver the selected menu when ordered, and such a function enables more accurate and diverse selections than the conventional system for ordering by phone.

However, such a technique, which is a conventional ordering system applied to a mobile device as it is, is not able to order and eat a desired food in a desired manner as much as desired, and thus cannot solve the above-described problems.

As related arts to the present invention, there have been disclosed Korean Registered Patent No. 10-1761741 (entitled 'BOMP-based food menu analysis recommendation system and method', registered on July 20, 2017), Korean Unexamined Patent Publication No. 10-2009-0124704 (entitled Method for standardizing food calorie and nutrient data using food recipes and information provision service system using the same', published on December 03, 2009), Korean Unexamined Patent Publication No. 10-2003-0017664 (entitled 'Group meal management system using the Internet', published on March 04, 2003), etc.

### Disclosure

### Technical Problem

To solve all the problems described above, one object of the present invention is to provide a modularized menu providing service system which provides a user device with menu configuration elements including modularized food elements and modularized setting elements, and transmits a menu configured according to the user's selection to a member store as an order. Accordingly, modularized food elements and setting elements can be combined such that food desired by the user can be ordered by a desired amount in a desired manner. In addition, even dishes or cooking tools can be selected by selecting setting elements such as packaging, tableware, and cooking utensils, thereby enabling the user to cook food as desired and to eat same.

Another object of the present invention is to provide a modularized menu providing service system which stores and manages standardized weights and standardized calories according to the unit amount of food, with regard to modularized food elements. Accordingly, the menu and calories of a menu can be provided in a practical manner by using the unit amount of food on the basis of a concept that can be used when a menu is actually provided. In addition, weights and calories are standardized and provided, which are difficult to accurately calculate when a menu is actually provided, thereby improving the level of satisfaction of users interested in diet and health.

### Technical Solution

To achieve the above-described objects, according to an aspect of the present invention,
there is provided a modularized menu providing service system which includes:
a service providing server that receives order information from a user device and transmits the order information to a member store device,
the service providing server includes a database configured to store and manage a menu configuration element including a modularized food element and a modularized setting element constituting a menu;
a menu configuration portion configured to provide the user device with the menu configuration element stored in the database and receive a selection of the provided menu configuration element from the user device to configure a menu;
a price calculation portion configured to calculate a price of the selected menu configuration element with regard to the configured menu; and
an ordering portion configured to receive order information from the user device and transmit the menu configured by the menu configuration module and the received order information to the member store device,
wherein the modularized food element includes a type of food, a unit amount of food configured according to the type of food, and a method for providing the food, and
the modularized setting element includes setting types including packaging, tableware, a cooking utensil, a heater, and kitchen cooking.

Preferably,
the database may store and manage a standardized weight and a standardized calorie according to the unit amount of food, with regard to the modularized food element, and
the service providing server may further include
an information calculation portion configured to calculate a standardized weight and a standardized calorie of a menu configured according to a selected menu configuration element by using the information stored in the database as the modularized food element provided by the menu configuration portion is selected by the user device.

Still preferably,
the database may store and manage a standardized volume according to the unit amount of food for the modularized food element, and
the information calculation portion may use information stored in the database to calculate the standardized volume of the menu according to a selected menu configuration element as the menu configuration element provided by the menu configuration portion is selected by the user device.

Still preferably,
the menu configuration portion may request the user device to modify the setting element according to the volume calculated by the information calculation portion.

Still preferably,
the database may to store and manage a standardized nutrient content according to the unit amount of food, with regard to the modularized food element, and
the information calculation portion may use information stored in the database to calculate the standardized nutrient content according to a selected food element as the modularized food element provided by the menu configuration portion is selected by the user device.

Preferably,
the database may store a unit price according to the unit amount of food for the modularized food element, and store a provision unit price for the modularized setting element, and
the price calculation portion may calculate a price according to a menu configuration element selected by the user device by using the unit price stored in the database.

Preferably, the service providing server may further include
a payment portion (160) configured to provide a payment process to the user device (200) for the price calculated by the price calculation portion.

Preferably, the order information may include
information on a selected member store and order time.

### Advantageous Effects

According to the modularized menu providing service system proposed by the present invention, the user device is provided with menu configuration elements including modularized food elements and modularized setting elements, and a menu configured according to the user's selection is transmitted to a member store as an order. Accordingly, modularized food elements and setting elements can be combined such that food desired by the user can be ordered by a desired amount in a desired manner. In addition, even dishes or cooking tools can be selected by selecting setting elements such as packaging, tableware, and cooking utensils, thereby enabling the user to cook food as desired and to eat same.

In addition, according to the present invention, standardized weights and standardized calories are stored and managed according to the unit amount of food, with regard to modularized food elements. Accordingly, the menu and calories of a menu can be provided in a practical manner by using the unit amount of food on the basis of a concept that can be used when a menu is actually provided. In addition, weights and calories are standardized and provided, which are difficult to accurately calculate when a menu is actually provided, thereby improving the level of satisfaction of users interested in diet and health.

### Description of Drawings

FIG. 1 is a diagram showing the configuration of a modularized menu providing service system according to an embodiment of the present invention.
FIG. 2 is a block diagram showing a detailed configuration of the service providing server 100 in a modularized menu providing service system according to an embodiment of the present invention.
FIG. 3 is a diagram showing an example of the user device provided with a menu configuration element in a modularized menu providing service system according to an embodiment of the present invention.
FIGS. 4 and 5 are diagrams illustrating examples of a modularized food element in a modularized menu providing service system according to an embodiment of the present invention.
FIG. 6 is a diagram illustrating an example of a modularized setting element in a modularized menu providing service system according to an embodiment of the present invention.
FIGS. 7 and 8 are diagrams illustrating an example of a menu configured by the menu configuration portion 120 in the modularized menu providing service system according to an embodiment of the present invention.

### Best Mode

### Mode for Invention

Hereinafter, various embodiments of the present invention will be described in detail with reference to the accompanying drawings, so that those skilled in the art can easily carry out the present invention. However, in the description of the embodiments of the present invention, a detailed description of well-known features or functions will be ruled out in order not to unnecessarily obscure the gist of the present disclosure. In addition, the same reference numerals will be assigned to elements having the same or similar functionality throughout the drawings.

In addition, it will be understood that when an element is referred to as being "connected" to another element in the specification, it may be directly connected or indirectly connected to another element.In addition, when some part is referred to 'include' some elements, unless explicitly described to the contrary, it means that other elements may be further included but not excluded.

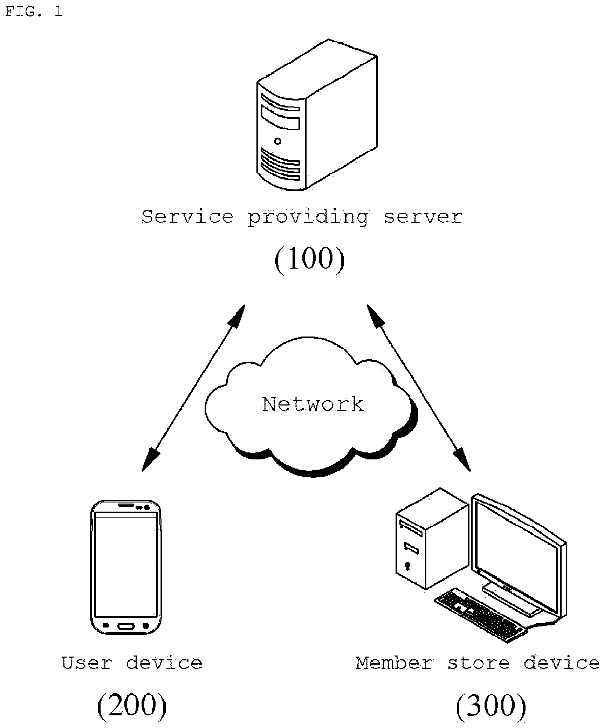

FIG. 1 is a diagram showing the configuration of a modularized menu providing service system according to an embodiment of the present invention. As shown in FIG. 1, a modularized menu providing service system according to an embodiment of the present invention may include a service providing server 100, and may further include a user device 200 and a member store device 300.

That is, according to the present invention, the service providing server 100 may provide a menu configuration element including a modularized food element and a modularized setting element to the user device 200, and transmit a menu configured according to a user's selection to the member store device 300 to place an order. The user may visit the member store and receive the ordered menu to eat or take out, and may make a delivery request to the user's location. Hereinafter, each component constituting the modularized menu providing service system according to an embodiment of the present invention will be described in detail.

The service providing server 100 may receive order information from the user device 200 and transmit the order information to the member store device 300. That is, while storing and managing a menu configuration element including a modularized food element and a setting element, the service providing server 100 may provide the menu configuration element to the user device 200 to receive an order through selection of modularized elements, and transmit the order information to the member store device 300 such that it is possible to smoothly place an order.

In this case, the user may search for a member store by using the location of the user device 200 or the location set by the user. However, the user may place an order with a member store through search or advertisement regardless of the location information. In addition, the service providing server 100 may receive an order time to receive a menu and seat reservation information from the user device 200 and transmit them to the member store device 300 together with the menu configuration information.

The user device 200, which is a device capable of transmitting and receiving data to and from the service providing server 100 through a network, may access to the service providing server 100 through an application or web to use the modularized menu providing service of the present invention.

The member store device 300, which is a device capable of transmitting and receiving data to and from the service providing server 100 through a network, may receive the menu configured by the user and order information from the service providing server 100. The member store may prepare a menu according to the received information and provide it to the user. In addition, when it is impossible to provide a menu, an order processing impossible message may be immediately transmitted to the service providing server 100.

In this case, the user device 200 and the member store device 300 may include a mobile device such as a smartphone, a tablet PC, a wearable device, and the like, and may include a personal computer (PC), a laptop computer, and the like. In addition, according to an embodiment, the member store device 300 may include a poster used in a member store. However, the user device 200 and the member store device 300 of the present invention are not limited to the types of devices described above, and a device that is capable of transmitting and receiving an order by transmitting and receiving data with the service providing server 100 through a network may be included in the user device 200 and the member store device 300 regardless of the specific type.

Meanwhile, in the present invention, the network may include a wired network such as a local area network (LAN), a wide area network (WAN), a value added network (VAN), and the like, and all types of wireless networks such as a satellite communication network, wireless broadband Internet (WIBRO), high speed downlink packet access (HSDPA), and the like.

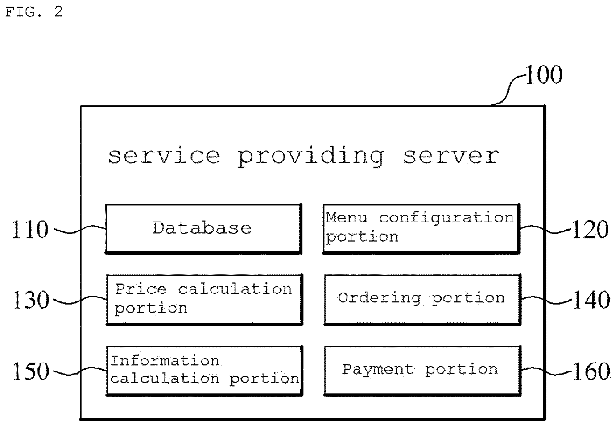

FIG. 2 is a block diagram showing a detailed configuration of the service providing server 100 in a modularized menu providing service system according to an embodiment of the present invention. As shown in FIG. 2, the service providing server 100 of the modularized menu providing service system according to an embodiment of the present invention may include a database 110, a menu configuration portion 120, a price calculation portion 130, and an ordering portion 140, and may further include an information calculation portion 150 and a payment portion 160.

The database 110 may store and manage a menu configuration element including a modularized food element and a modularized setting element constituting a menu. In this case, the modularized food element may include a type of food, and a unit amount of food and a provision method set according to the type of food, and the modularized setting element may include a type of setting including packaging, tableware, cooker, heater, and kitchen cooking.

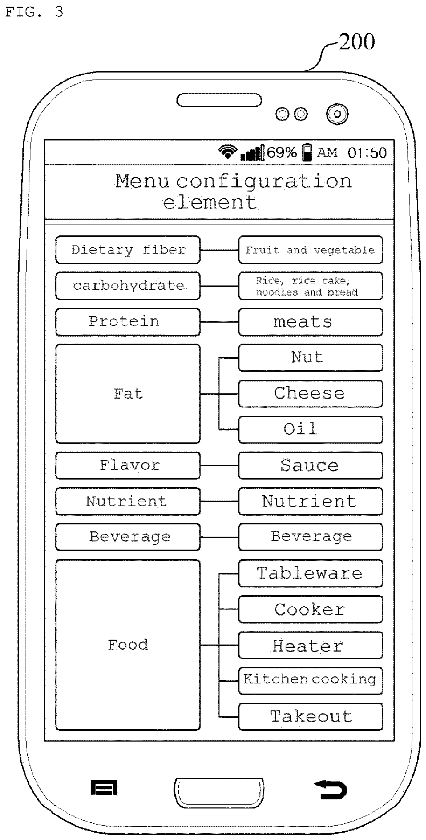

FIG. 3 is a diagram showing an example of the user device 200 provided with a menu configuration element in a modularized menu providing service system according to an embodiment of the present invention. In the modularized menu providing service system according to an embodiment of the present invention, as shown in FIG. 3, the database 110 may modularize information about various types of food included in large classification of dietary fiber, carbohydrates, proteins, fats, flavors, nutrients and beverages, and store and manage the modularized food element. In addition, as shown in FIG. 3, by modularizing setting information related to food provision or cooking, such as a dish, a cooker, a heater, kitchen cooking, takeout, etc., included in the large classification of 'food', the modularized setting element may be stored and managed.

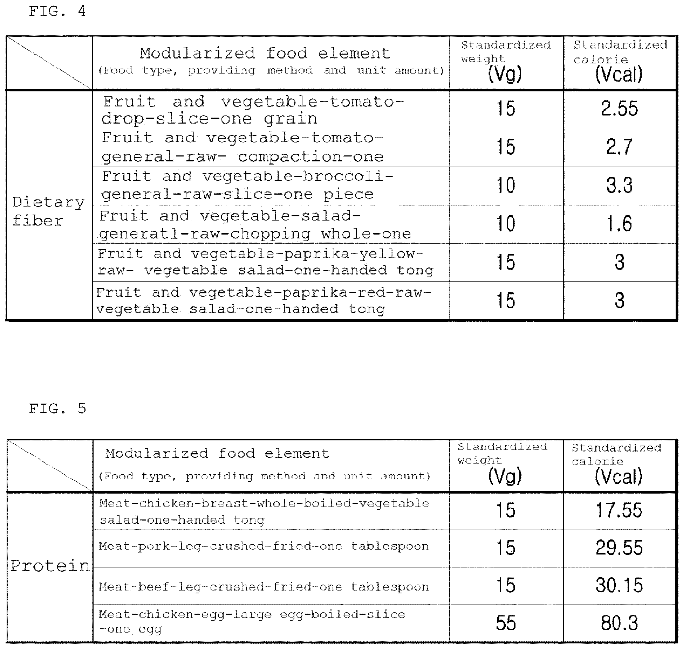

FIGS. 4 and 5 are diagrams illustrating examples of a modularized food element in a modularized menu providing service system according to an embodiment of the present invention. FIG. 4 shows a food using cod meal as dietary fiber as an example, and Fig. 5 shows a food product using cod meal as protein as an example. The modularized food element stored in the database 110 may include a type of food, and a unit amount of food and a provision method set according to the type of food. That is, referring to the example of the second row of FIG. 4, the corresponding food element may be a general tomato among fruits, the food providing method may be compaction in raw, and the unit amount may be one tablespoon. In addition, referring to the example of the first row of FIG. 5, as the corresponding food element, the type of food may be chicken breast among meat, the method of providing food may be a method of boiling and providing food as a whole, and the unit amount may be one-handed tongs (amount picked with one hand). As described above, the method of providing food may vary according to the type of food, and the unit amount of food may vary according to the type of food and the method of providing food. Particularly, in the present invention, by using a unit amount of food that can be used when providing or cooking actual food, the amount may be intuitively understood and practical.

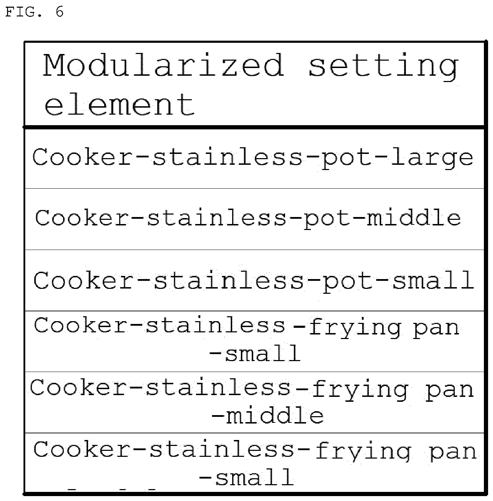

FIG. 6 is a diagram illustrating an example of a modularized setting element in a modularized menu providing service system according to an embodiment of the present invention. As shown in FIGS. 3 and 6, in the modularized menu providing service system according to an embodiment of the present invention, the modularized setting element may include a setting type such as a dish, a cooker, a heater, kitchen cooking, takeout (packaging containers), etc., and a material or size that is selectable for each setting type. That is, in the example shown in FIG. 6, the setting type may include a combined setting element of items such as a port or a flying pen as a cooker that means a bowl used when cooking, stainless steel, a large/medium/small size, etc.

In addition, the database 110 may store and manage a standardized weight and a standardized calorie according to the unit amount of food for the modularized food element. That is, as shown in FIGS. 4 and 5, the unit amounts of food may be different for each food element, and the database 110 may store and manage the standardized weight and standardized calorie according to the unit amount of food, respectively. In this case, the standardized weight and standardized calories may mean the average weight and calories of a food element. In the present invention, since practical units such as tablespoon, one piece, and one-hand tongs are used as the unit amount of food instead of a unit of weight or volume, specific weights and calories may be different for each providing. Therefore, by storing and managing the average weight and calories provided with one tablespoon, one piece, and one-hand tongs as standardized weight and standardized calories, it is possible to infer the actual weight and calories while maintaining convenience. Referring to the example in the second row of FIG. 4, the standardized weight of one tablespoon of raw chopped tomatoes, which is a corresponding food element, may be 15 grams, and the standardized calories may be 2.7 kilocalories.

Meanwhile, the database 110 may store a unit price according to the unit amount of food for a modularized food element, and store a provision unit price for a modularized setting element. For example, the unit price of a tablespoon of raw chopped tomatoes may be stored. With regard to the modularized setting element, the unit price of tableware, a cooker, a heater, etc. provided by default may be '0', but in the case of a special setting element, the unit price may be calculated and stored. The unit prices may be different depending on the material or size of a packaging container. In addition, the database 110 may store and manage the nutrient content, volume, and origin of the modularized food element.

The menu configuration portion 120 may provide the user device 200 with the menu configuration element stored in the database 110 and receive the selection of the menu configuration element provided from the user device 200 to construct the menu. That is, the user device 200 may access to the service providing server 100 by using a web or an application, and the menu configuration portion 120 may provide a menu configuration as shown in FIG. 3. When the user selects "fruits and vegetables" on the screen as shown in FIG. 3, the modularized food elements as shown in FIG. 4 may be selected in sequence of "Tomato -> General -> Raw - > Compaction -> One tablespoon". In this case, the menu configuration portion 120 may receive an input by adjusting the quantity of the modularized food elements to the number of unit amounts of food. For example, the user may input three tablespoons by adjusting the quantity of one tablespoon of normal raw chopped tomato.

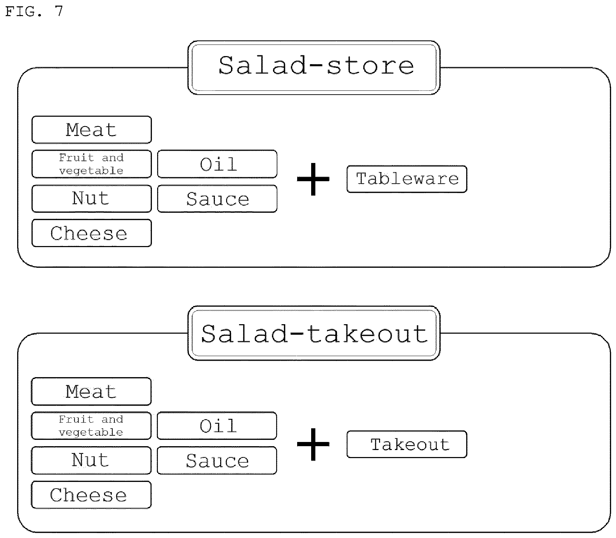

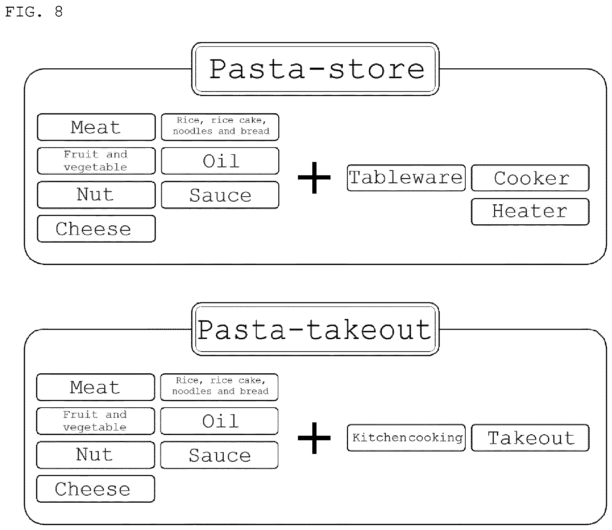

FIGS. 7 and 8 are diagrams illustrating examples of a menu configured by the menu configuration portion 120 in the modularized menu providing service system according to an embodiment of the present invention. As shown in FIG. 7, in the modularized menu providing service system according to an embodiment of the present invention, a user may select the modularized food elements and quantities included in meats, fruits and vegetables, nuts, cheeses, oils, and sauces to suit his tastes, and select a desired tableware from among the modularized setting elements, such as a medium-sized glass bowl, thereby constructing a salad for consumption by visiting a member store. In addition, it is possible to construct a salad for package purchase or delivery by selecting the same food element and then selecting a take-out container from the modularized setting elements.

Meanwhile, as shown in FIG. 8, in the modularized menu providing service system according to an embodiment of the present invention, the user may select a modularized food element included in meats, fruits and vegetables, nuts, cheeses, rice cake noodles, oils and sauces to suit his tastes, and the quantity, and may select a tableware, a cooker, and a heater from the modularized setting elements to construct a menu. In this case, the user may visit the member store, and use the cooker (frying pan) and heater (induction) selected as setting elements to cook directly with the selected food elements and place it on a tableware (plate) to eat. In addition, when the same modularized food elements are selected and kitchen cooking and takeout are selected among the modularized setting elements, instead of cooking directly, the menu cooked in the kitchen with the selected food elements may be placed in a container for takeout to purchase it.

As shown in FIGS. 7 and 8, in the modularized menu providing service system according to an embodiment of the present invention, the user may receive a desired set menu by selecting food ingredients and amounts as desired, as well as direct cooking or selecting a packaging container.

Meanwhile, according to an embodiment, the menu configuration portion 120 may configure various menus such as salad, pasta, bibimbap, naengmyeon, curry rice, etc., in advance by using modularized food elements and modularized setting elements, and provide the pre-configured menu to the user device 200 (coordination menu). The user may modify the menu to a desired menu by adding, deleting, or changing the modularized food element and the modularized setting element included in the pre-configured menu. In this case, the pre-configured menu may be a menu that user prefer in advance, but may be a menu that has been previously ordered by the user and extracted from the user's order history information. In the modularized menu providing service system according to an embodiment of the present invention, by including such a configuration, it is possible to help users who experience difficulty in configuring a menu, provide a guide, and improve user convenience.

The price calculation portion 130 may calculate a price according to the selected menu configuration element for the configured menu. In more detail, the price calculation portion 130 may calculate a price according to the menu configuration element selected by the user device 200 by using the unit price stored in the database 110. For example, referring to the example of the second row of FIG. 4, when one tablespoon of raw chopped tomatoes is 100 won, and the quantity of the corresponding food element is '3', the price may be calculated as 300 won. By using the unit price and quantity stored in all the menu configuration elements, the price calculation portion 130 may calculate the total price of the corresponding menu.

Meanwhile, in the modularized menu providing service system according to an embodiment of the present invention, since the database 110 stores and manages the unit price according to the unit amount of food, the price calculation portion 130 may calculate the price according to the unit amount, not the actual weight of the menu. In addition, the price calculation portion 130 may calculate and provide the price each time a menu configuration element is selected by the user device 200, so that the user may configure the menu while monitoring the price.

The ordering portion 140 may receive order information from the user device 200 and transmit the menu configured in a menu configuration module and the received order information to the member store device 300. In this case, the order information may include information about the selected member store and order time, and may further include whether a seat is reserved. In this case, the order time information may be a time at which a menu is to be provided, or may be an order time according to an embodiment. In the member store that has received order information and information on a configured menu from the ordering portion 140, a menu may be prepared to be provided to a user according to order time information according to food elements and setting elements constituting the menu.

The information calculation portion 150 may calculate the standardized weight and standardized calories of the menu that is configured according to the menu configuration element selected by using the information stored in the database 110 as the modularized food element provided by the menu configuration portion 120 is selected by the user device 200. Since the database 110 stores and manages the standardized weight and standardized calorie according to the unit amount of food, for the modularized food element, the standardized weight and calorie of the menu calculated by the information calculation portion 150 may be calculated according to the unit amount such as one tablespoon or one-hand tongs. For example, when the menu is a chicken breast salad, the standardized weight calculated by the information calculation portion 150 may be 400 grams and the standardized calorie may be 200 kilocalories, whereas the weight of the actual chicken breast salad may be 410 grams and the calorie may be 210 kilocalories. In the present invention, the information calculation portion 150 may calculate the standardized weight and calories of the menu to provide them to the user device 200, so that the user may predict the amount or calories of the menu within an error range while using a practical unit amount, so it may be used for health care.

In addition, the database 110 may store and manage the standardized volume according to the unit amount of food for the modularized food element, and the information calculation portion 150 may calculate the standardized volume of a menu according to the menu configuration element selected by using the information stored in the database 119 as the menu configuration element provided from the menu configuration portion 120 is selected by the user device 200. That is, the information calculation portion 150 may calculate the standardized volume of a menu and provide it to the user device 200, similar to the calculation of the above-described standardized weight and calories. However, since the volume of a menu may be different according to the cooking method, even if the same food element and quantity are selected, the volume of the menu calculated according to the modularized setting element may be different.

The menu configuration portion 120 may request the user device 200 to modify a setting element according to the volume calculated by the information calculation portion 150. For example, when a small-sized packaging container is selected as a setting element while ordering a chicken breast salad, the sum of the selected food elements may excessively exceed the capacity of the corresponding packaging container. As described above, when the capacity of the setting element exceeds a predetermined volume or more, the menu configuration portion 120 may request the user device 200 to modify the setting element to smoothly provide the menu.

Meanwhile, the database 110 may store and manage the standardized nutrient content according to the unit amount of food with respect to the modularized food element, and the information calculation portion 150 may calculate the standardized nutrient content of a menu according to the selected food element by using the information stored in the database 110 as the modularized food element provided by the menu configuration portion 120 is selected by the user device 200. That is, the information calculation portion 150 may calculate the standardized nutrient content of the menu and provide it to the user device 200, similar to the calculation of the above-described standardized weight and calories. For example, by calculating the amounts of dietary fiber, carbohydrate, protein and fat of the menu and providing the amounts to the user device 200, the user may check the nutrients included in the menu to be consumed and make an effort to eat a balanced meal. However, since the information calculation portion 150 of the present invention provides the nutrient content of the menu calculated by using the standardized nutrient content according to the unit amount of the practical food, it may be different from the actual nutrient content provided by the menu.

As described above, information about the standardized weight, standardized calorie, and standardized nutrient content calculated by the information calculation portion 150 may be provided to the user device when ordering, and when a menu is provided to an member store, the receipt may be provided on the outer surface of the packaging container, etc. According to an embodiment, it is possible to help the user understand by displaying and providing the actual weight of the menu together.

The payment portion 160 may provide a payment process to the user device 200 for the price calculated by the price calculation portion 130. In this case, the payment portion 160 may provide a payment process according to various payment methods usable on online and mobile, such as credit card, account transfer, coupon, point, mobile payment, etc.

As described above, according to the modularized menu providing service system proposed by the present invention, the service providing server 100 provides the user device 200 with menu configuration elements including modularized food elements and modularized setting elements, and transmits the menu configured according to the user's selection to a member store as an order. Accordingly, modularized food elements and setting elements can be combined such that food desired by the user can be ordered by a desired amount in a desired manner. In addition, even dishes or cooking tools can be selected by selecting setting elements such as packaging, tableware, and cooking utensils, thereby enabling the user to cook food as desired and to eat same. In addition, the standardized weights and standardized calories are stored and managed according to the unit amount of food, with regard to modularized food elements. Accordingly, the menu and calories of a menu can be provided in a practical manner by using the unit amount of food on the basis of a concept that can be used when a menu is actually provided. In addition, weights and calories are standardized and provided, which are difficult to accurately calculate when a menu is actually provided, thereby improving the level of satisfaction of users interested in diet and health.

As described above, the present invention should be understood that numerous other modifications and embodiments can be devised by those skilled in the art, and the spirit and scope of the principles of the present invention will fall within the scope of the appended claims.

### [Description of Reference Numeral]

100: Service providing server
110: Database
120: Menu configuration portion
130: Price calculation portion
140: Ordering portion
150: Information calculation portion
160: Payment portion
200: User device
300: Member store device

## Claims

1. A modularized menu providing service system comprising:
a service providing server (100) that receives order information from a user device (200) and transmits the order information to a member store device (300), wherein the service providing server includes:
a database (110) configured to store and manage a menu configuration element including a modularized food element and a modularized setting element constituting a menu; a menu configuration portion (120) configured to provide the user device (200) with the menu configuration element stored in the database (110) and receive a selection of the provided menu configuration element from the user device (200) to configure a menu; a price calculation portion (130) configured to calculate a price of the selected menu configuration element with regard to the configured menu; and an ordering portion (140) configured to receive order information from the user device (200) and transmit the menu configured by the menu configuration module and the received order information to the member store device (330), wherein the modularized food element includes a type of food, a unit amount of food configured according to the type of food, and a method for providing the food, and the modularized setting element includes setting types including packaging, tableware, a cooking utensil, a heater, and kitchen cooking.

2. The modularized menu providing service system of claim 1, wherein the database (110) is configured to store and manage a standardized weight and a standardized calorie depending on the unit amount of food, with regard to the modularized food element, and the service providing server (100) further includes an information calculation portion (150) configured to calculate a standardized weight and a standardized calorie of a menu configured according to a selected menu configuration element by using the information stored in the database (110) as the modularized food element provided by the menu configuration portion (120) is selected by the user device (200).

3. The modularized menu providing service system of claim 2, wherein the database (110) is configured to store and manage a standardized volume according to the unit amount of food for the modularized food element, and the information calculation portion (150) is configured to use information stored in the database (110) to calculate the standardized volume of the menu according to a selected menu configuration element as the menu configuration element provided by the menu configuration portion (120) is selected by the user device (200).

4. The modularized menu providing service system of claim 3, wherein the menu configuration portion (120) is configured to request the user device (200) to modify the setting element depending on the volume calculated by the information calculation portion (150).

5. The modularized menu providing service system of claim 2, wherein the database (110) is configured to store and manage a standardized nutrient content depending on the unit amount of food, with regard to the modularized food element, and the information calculation portion (150) is configured to use information stored in the database (110) to calculate the standardized nutrient content depending on a selected food element as the modularized food element provided by the menu configuration portion (120) is selected by the user device (200).

6. The modularized menu providing service system of claim 1, wherein the database (110) is configured to store a unit price according to the unit amount of food for the modularized food element, and store a provision unit price for the modularized setting element, and the price calculation portion (130) is configured to calculate a price according to a menu configuration element selected by the user device (200) by using the unit price stored in the database (110).

7. The modularized menu providing service system of claim 1, wherein the service providing server (100) further includes a payment portion (160) configured to provide a payment process to the user device (200) for the price calculated by the price calculation portion (130).

8. The modularized menu providing service system of claim 1, wherein the order information includes information on a selected member store and order time.
